# EUROPEAN PATENT SPECIFICATION

(11) **EP 3 684 355 B1**
(45) Date of publication and mention of the grant of the patent: **08.09.2021**
(21) Application number: 18782202.8
(22) Date of filing: 17.09.2018
(51) Int. Cl.: A61K 31/4172, A61K 31/4178, A61P 29/00, A61P 3/10

(54) **OVOTHIOLS FOR THE TREATMENT OF CHRONIC LOW GRADE SYSTEMIC INFLAMMATION (CLGSI) AND RELATED DISEASES**
OVOTIOLE ZUR BEHANDLUNG VON CHRONISCHEN MINDERWERTIGEN SYSTEMISCHEN ENTZÜNDUNGEN (CLGSI) UND VERWANDTEN ERKRANKUNGEN
OVOTHIOLS POUR LE TRAITEMENT D'UNE INFLAMMATION GÉNÉRALISÉE CHRONIQUE DE FAIBLE GRADE (CLGSI) ET DE MALADIES ASSOCIÉES

(30) Priority: 19.09.2017 IT 201700104529
(43) Date of publication of application: 29.07.2020
(73) Proprietor: Universita' Degli Studi "G. d'Annunzio" Chieti-Pescara, 66100 Chieti (IT); Stazione Zoologica "Anton Dohrn", 80121 Napoli (IT)
(72) Inventor: PALUMBO, Anna, 80121 Napoli (IT); PANDOLFI, Assunta, 66100 Chieti (IT); CASTELLANO, Immacolata, 80121 Napoli (IT); DI TOMO, Pamela, 66100 Chieti (IT)
(74) Representative: Currado, Luisa
(86) International application number: PCT/IB2018/057098
(87) International publication number: WO 2019/058247

(56) References cited:
- WO-A2-2007/062343
- US-A- 4 898 878
- PHILIP C. CALDER ET AL: "Dietary factors and low-grade inflammation in relation to overweight and obesity", BRITISH JOURNAL OF NUTRITION, vol. 106, no. S3, 1 December 2011 (2011-12-01), pages S5-S78, XP055479408, UK ISSN: 0007-1145, DOI: 10.1017/S0007114511005460
- IMMACOLATA CASTELLANO ET AL: "Anti-Inflammatory Activity of Marine Ovothiol A in an In Vitro Model of Endothelial Dysfunction Induced by Hyperglycemia", OXIDATIVE MEDICINE AND CELLULAR LONGEVITY, vol. 2018, 1 January 2018 (2018-01-01), pages 1-12, XP055479475, US ISSN: 1942-0900, DOI: 10.1155/2018/2087373

## Description

### Background of the invention

The present invention relates to the pharmaceutical sector as it concerns the use of ovothiol for the treatment of Chronic Low-grade Systemic Inflammation (CLGSI) and related pathologies.

### Prior art

Ovothiol is a metyl-5-thioistidine isolated from the eggs of various echinoderms. Three forms have been characterized, ovothiol A, B and C. Ovothiol A was isolated and then characterized from the eggs of the sea urchin *Paracentrotus lividus* (Palumbo, A., d'Ischia, M., Misuraca, G., Prota, G. (1982) Isolation and structure of a new sulphur containing aminoacid from sea urchin eggs. Tetrahedron Lett., 23, 3207-3208; Palumbo, A., Castellano, I., Napolitano, A. (2017) Ovothiol: a potent natural antioxidant from marine organisms. In Blue Biotechnology. Production and use of marine molecules. PART 2: MARINE MOLECULES FOR DISEASE TREATMENT / PREVENTION AND FOR BIOLOGICAL RESEARCH. Edited by Stephane La Barre, Stephen S. Bates, Wiley, pp. 583-610).

Ovothiol B was isolated from the scallop *Chlamys hastata* (Turner, E., Klevit, R., Hager, L.J., Shapiro, BM. (1987) Ovothiols, a family of redox-active mercaptohistidine compounds from marine invertebrate eggs. Biochemistry, 26, 4028-4036) and ovothiol C from the eggs of the sea urchins *Sphaerechinus granularis* (Palumbo, A., Misuraca. G., d'Ischia, M., Donaudy, F., Prota, G. (1984) Isolation and distribution of 1-methyl-5-thiol-L-histidine disulphide and a related metabolite in eggs from echinoderms. Comp. Biochem. Physiol., 78B, 81-83) and *Strongylocentrotus purpuratus* (Turner, E., Klevit, R., Hopkins, P.B., Shapiro, B.M. (1986) Ovothiol: a novel thiohistidine compound from sea urchin eggs that confers NAD(P)H-02 oxidoreductase activity on ovoperoxidase. J. Biol. Chem., 261, 13056-13063).

Ovothiol A has been recently identified in the green microalga *Euglena gracilis* (O'Neill, E.C., Trick, M., Hill, L., Rejzek, M., Dusi, R.G., Hamilton, C.J., Zimba, P.V., Henrissat, B., Field, R.A. (2015) The transcriptome of Euglena gracilis reveals unexpected metabolic capabilities for carbohydrate and natural product biochemistry. Mol. Biosyst., 11, 2808-2820).

The class of ovothiols shows antioxidant properties, thanks to the peculiar position of the thiol group on the imidazole ring of histidine (Holler, T.P. and Hopkins, P.B. (1988) Ovothiols as biological antioxidants. The thiol groups of ovothiol and glutathione are chemically distinct. J. Am. Chem. Soc., 110, 4837-4838). In particolar, ovothiols play a key role in controlling the cellular redox status since they are able to react with glutathione (Shapiro, B.M., Turner, E.E., Hopkins, P.B., Klevit, R.E., Holler, T.P., Spaltenstein, A. (1990) Antioxidant Thiohistidine Compounds. Patent US 4898878 A ; Jacob, C. (2006) A scent of therapy: pharmacological implications of natural products containing redox-active sulfur atoms. Nat. Prod. Rep., 23, 851-863).

In sea urchin the role of ovothiol A has been associated to detoxification from peroxides produced during fertilization (Turner, E., Hager, L.J., Shapiro, B.M. (1988) Ovothiol replaces glutathione peroxidase as a hydrogen peroxide scavenger in sea urchin eggs. Science, 242, 939-941), and as response to environmental stress during development of embryos in sea water (Castellano, I., Migliaccio, O., D'Aniello, S., Merlino, A., Napolitano, A., Palumbo, A. (2016) Shedding light on ovothiol biosynthesis in marine metazoans. Sci. Rep., 6, 21506) .

Regarding the biological activities, an ovothiol analogue has been shown to possess neuro-protective activity by regulation of redox homeostasis (Vamecq, J., Maurois, P., Bac, P., Bailly, F., Bernier, J.L., Stables, J.P., Husson, I., Gressens, P. (2003) Potent mammalian cerebroprotection and neuronal cell death inhibition are afforded by a synthetic antioxidant analogue of marine invertebrate cell protectant ovothiols. Eur. J. Neurosci., 18, 1110-1120).

Moreover, it is known that ovothiol A induces autophagy in epatocarcinoma cell lines, without inducing a reduction of reactive oxygen species (Russo, G.L., Russo, M., Castellano, I., Napolitano, A., Palumbo, A. (2014) Ovothiol isolated from sea urchin oocytes induces autophagy in the Hep-G2 cell line. Mar. Drugs, 12, 4069-4085).

At present, a synthetic chemical procedure for the synthesis of ovothiol and its derivatives is available, which is is very expensive in terms of costs and time and not eco-compatible (Holler, T.P., Ruan, F., Spaltenstein, A., Hopkins, P.B. (1989) Total synthesis of marine mercaptohistidines: ovothiols A, B, and C. J. Org. Chem., 54, 4570-4575). This synthesis has been recently modified, but although faster it is not very reproducible (Mirzahosseini, A., Hosztafi, S., Tóth, G., Noszál, B. (2014) A cost-effective synthesis of enantiopure ovothiol A from L-histidine, its natural precursor. Arkivoc (vi) 1-9). Recently, an alternative organic synthesis has been reported which leads to the formation of 5-thio-histidine, the unmethylated precursor of oviolanol A (Daunay, S., Lebel, R., Farescour, L., Jean-Claude Yadan, J.-C., Erdelmeier I. (2016) Short protecting-group-free synthesis of 5-acetylsulfanylhistidines in water: novel precursors of 5-sulfanyl-histidine and its analogues. Org. Biomol. Chem., 14, 10473-10480).

Chronic Low-grade Systemic Inflammation (CLGSI) plays a crucial role in the development and progression of a large number of diseases such as diabetes, cardiovascular diseases, numerous cancers and all degenerative diseases, which have this common denominator. It is a subtle pathological condition, sometimes hidden for years, with important consequences and considerable unexpected frequency.

The specific causes of CLGSI onset may be mainly found in environmental, food, and in general in lifestyle-related factors.

In particular, they are responsible for the onset of chronic low-grade systemic inflammation (CLGSI): the excessive consumption of saturated fats of animal and vegetable origin, trans fatty acids of industrial origin and carbohydrates with a high glycemic index, the low intake of long-chain polyunsaturated fat acids, omega 3 type, of vegetable fibers and fruit and vegetables, the altered distribution of food intake during the day not in line with the physiological metabolic-hormonal circadian cycles, chronic stress, the lack or the excess of physical activity, the sleep deficit, or the displacement of the falling asleep to non-physiological and late hours, environmental pollution, cigarette smoking, infections and acute inflammations partially resolved or too rapid for the use of suppressive pharmacological therapies.

Among the factors that trigger the Chronic Low Degree Systemic Inflammation CLGSI) is also chronic stress of different origins such as metabolic stress due to poor nutrition, chronic diseases, and use of drugs; physical stress due to sedentary lifestyle and sarcopenia, sports overload, heavy work, reduction of sleep hours, increase in age; emotional stress caused by traumas and accidents, grief, physical or mental abuse, reduction of socio-economic status, divergences between common and social values, hereditary factors or acquired during the fetal period.

Therefore the chronic stress causes the reduction of the muscular mass, the activation of the hypothalamus-hypophysis-adrenal hormonal axis and of the sympathetic nervous system. This leads to an increase in the values of cortisol and angiotensin, adrenaline and noradrenaline, blood glucose with hyper-insulinemia and insulin resistance and subsequent development of type 2 diabetes mellitus; the increase in adipose tissue with hypertrophy-hyperplasia of adipose cells that causes the recruitment of macrophages and the production of pro-inflammatory factors, such as interleukins IL2, IL6 and the Tumor Necrosis Factor alpha (TNFα).

The combination of overweight or obesity and hyperglycaemia, arterial hypertension and dyslipidemia leads to an increased risk of cardio-vascular diseases such as, for example, myocardial infarction, stroke, chronic cerebral ischemia. It also determines the increase in thyroid hormones, the reduction of antibodies, testosterone, and growth hormone (GH) and vitamins of the B type.

Further it is responsible for the tendency to develop a metabolic acidosis, which is promptly neutralized by the bone system with the consumption of phosphates, calcium, magnesium and the onset of osteoporosis; by the blood-albumin, renal and respiratory systems.

There are also alterations in body composition such as reduction of total body water, calcium, magnesium, and potassium, and increase of sodium, with consequent increase or loss of weight, depending on the alterations present. Finally, an anomalous development of the bacterial flora of the oral, gingival and intestinal cavities can occur.

The persistence of the condition of Chronic Low-grade Systemic Inflammation (CLGSI) can cause the onset of related diseases: chronic inflammatory diseases, such as rheumatoid arthritis, polymyalgia rheumatica, fibromyalgia, chronic fatigue syndrome and psoriatic arthritis; autoimmune diseases, such as atopic dermatitis, psoriasis and allergic conjunctivitis; chronic degenerative diseases, such as multiple sclerosis, Parkinson's disease, amyotrophic lateral sclerosis (ALS) and Alzheimer's disease; tunours, such as breast, prostate and colorectal cancers; obesity; diabetes; arterial hypertension; hypercholesterolemia; cardiovascular diseases, such as myocardial infarction and stroke; osteoporosis; diseases of the respiratory apparatus, such as allergic rhinitis and asthma; and polycystic ovary syndrome.

Philip C. Calder ET AL: "Dietary factors and low-grade inflammation in relation to overweight and obesity", British Journal of Nutrition, vol. 106, no. S3, 1 December 2011 (2011-12-01), pages S5-S78 discloses the use of antioxidants such as vitamin C, vitamin E and carotenoids against chronic low-grade systemic inflammation.

At present, diseases associated with oxidative stress and related to cardio- and cerebro-vascular diseases are of particular interest. Among these the most widespread is diabetes mellitus which is associated with the onset of plasma oxidative stress and chronic vascular inflammation, which are the main alterations underlying the development of diabetes-associated cardiovascular diseases (Pandolfi, A., De Filippis, EA (2007) Chronic hyperglycemia and nitric oxide bioavailability play a pivotal role in pro-atherogenic vascular modifications, Genes Nutr. 2, 195-208). In particular, endothelial dysfunction is the main cause of cardiovascular disease development and is linked to a greater expression of endothelium adhesion molecules and to the infiltration of monocytes in the intima, two events that are crucial in the promotion of atherosclerosis.

Also nitric oxide (NO), which is constantly produced by endothelial cells, plays an important role in the maintenance of vascular homeostasis and in the pro-inflammatory response that characterizes the initial phases of atherosclerosis. Endothelial bioavailability of NO is therefore considered beneficial for endothelial functions and more generally for vascular health.

### Technical problen

In the pharmaceutical field there is a lot of attention in the research of active ingredients of natural origin to be used as food supplements in the prevention of the onset of inflammatory diseases and as medicaments for the treatment of the same pathologies.

It is important that the active ingredients are available in large quantities and can be used through easy application, high yield and low cost.

The inventors of the present invention have surprisingly found that ovothiol, upon incorporation into endothelial cells, acts on the endogenous mechanism of inflammation, which is mainly regulated by the inflammatory cytokines. In particular, the inventors have shown that the pathway triggered by the inflammatory cytokine TNFα is inhibited by incubation with ovothiol in endothelial cultures from the umbilical cord of women with gestational diabetes. The mechanism of action of ovothiol on the inflammatory process differs from that of the induction of autophagy in hepatocellular carcinoma cells because in the last case ovothiol does not act on cytokines but induces the expression of specific molecular autophagic markers independently from the ability of the molecule to reduce reactive oxygen species.

Moreover, the inventors of the present application have extracted ovothiol from the eggs of P. *lividus* in large amounts. The sea urchin can be bred, without depleting marine resources.

### Object of the invention

With reference to the attached claims, the technical problem is therefore solved by providing
1. Compounds of formula (I): 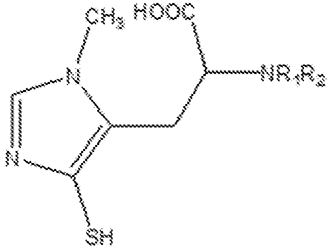 or of formula (II) 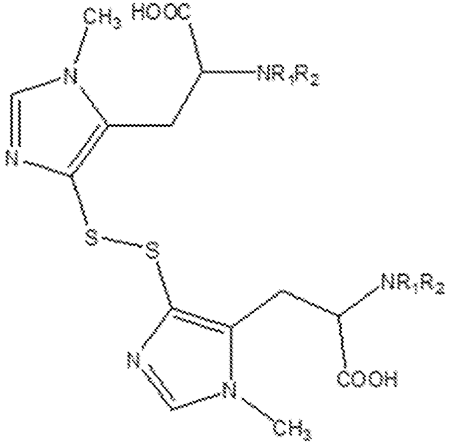
wherein R₁ and R₂, the same or different, are H or CH₃
for the prevention and the treatment of Chronic low-grade systemic inflammation (CLGSI) associated with the diseases related to Chronic low-grade systemic inflammation (CLGSI) selected from the group consisting of: chronic inflammatory diseases, autoimmune diseases, chronic degenerative diseases, tumours related to Chronic low-grade systemic inflammation (CLGSI), obesity, diabetes, arterial hypertension, osteoporosis, diseases of the respiratory apparatus and polycystic ovary syndrome wherein the compounds of formula (I) and (II) reduce chronic low-grade systemic inflammation.

### Brief description of the figures

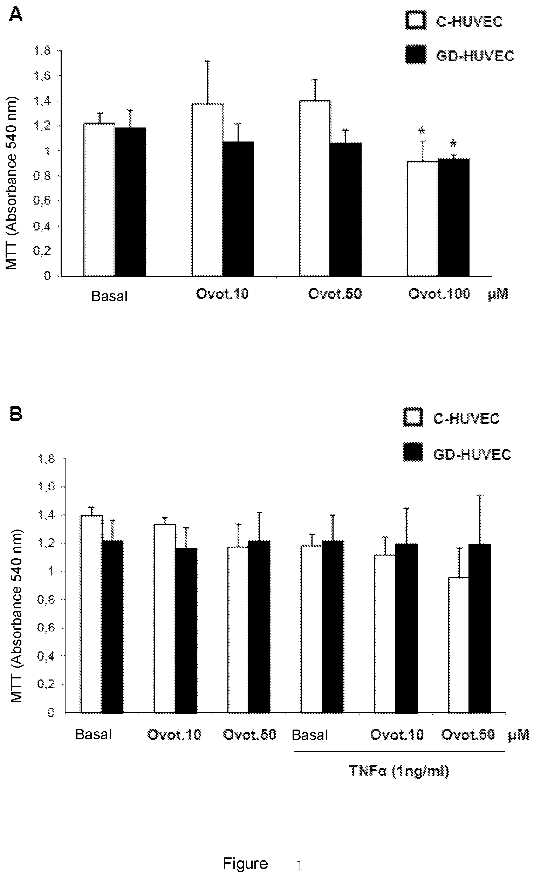

Figure 1 shows the histogram, panel A, of the results of a MTT assay on the viability of the human umbilical cord endothelial cells (Human Umbilical Cord Endothelial Cells - HUVEC) control (C-HUVEC) and cells extracted from umbilical cord of women with Gestational Diabetes (GD-HUVEC) in the presence of increasing concentrations of ovotain A; panel B, the results of an MTT assay on the viability of C-HUVEC and GD-HUVEC in the presence of increasing concentrations of ovothiol A in the presence and absence of TNFα (ANOVA test: * p <0.05 vs Basal).

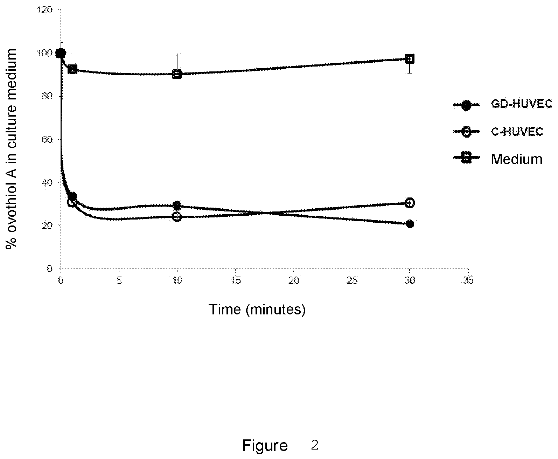

Figure 2 shows the graph of the results of the analysis by high-pressure liquid chromatography (HPLC) of the bioavailability of ovothiol A in the culture medium of C- and GD-HUVEC cells treated with 50 µM ovothiol.

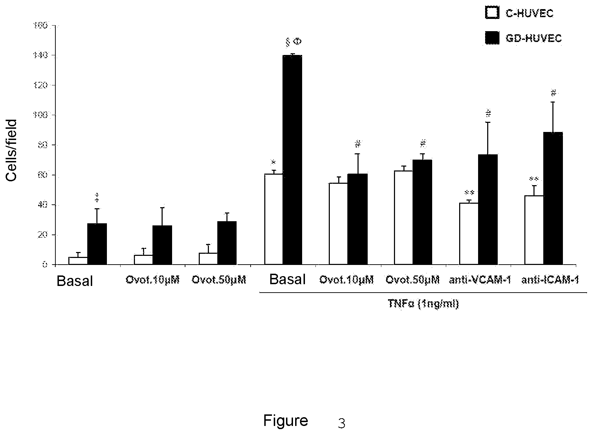

Figure 3 shows the histogram of the results of the adhesion of the human monocytes (U937 cells) in C- and GD-HUVEC (number of adherent cells / field) in the presence and absence of ovothiol A, stimulated or not with TNFα, and treated with antibodies anti- VCAM-1 and anti-ICAM-1 (ANOVA test: *p<0.05 vs Basal C-HUVEC; **p<0.05 vs TNFα C-HUVEC, §p<0.05 vs Basal GD-HUVEC, #p<0.05 vs TNFα GD-HUVEC) T-Test: 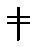 p<0.01 Basal GD-HUVEC vs Basal C-HUVEC, Φp<0.00001 TNFα GD-HUVEC vs TNFα C-HUVEC).

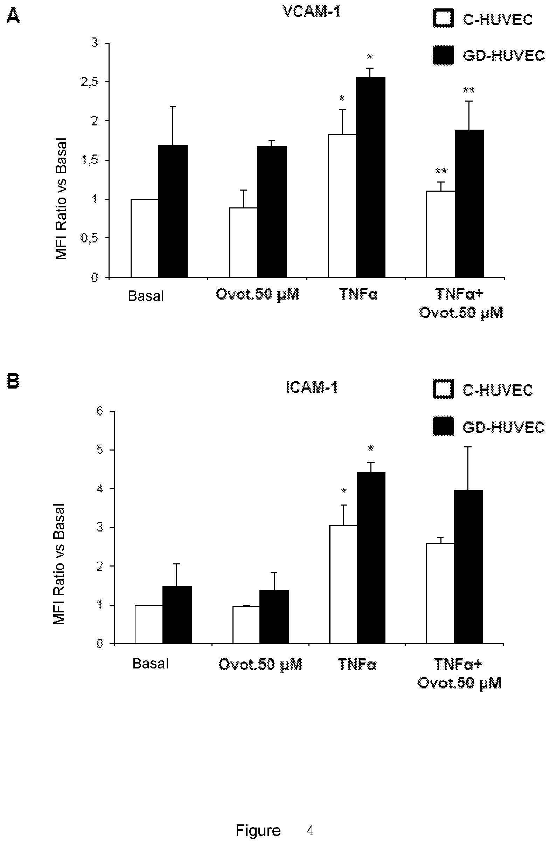

Figure 4 shows the histogram of the results obtained by flow cytometry of the exposure of the molecules VCAM-1 (panel A) and ICAM-1 (panel B) on the surface of C-HUVEC and GD-HUVEC cells treated and not treated with 50 µM of ovothiol A, in the presence or absence of TNFα (ANOVA test: *p<0.05 vs Basal, **p<0.05 vs TNFα).

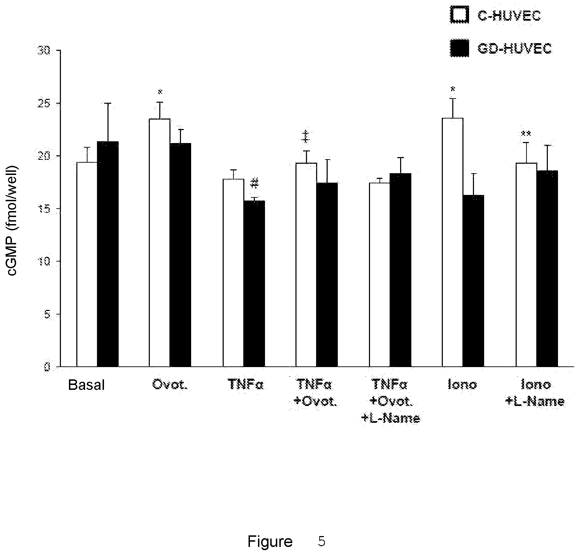

Figure 5 shows the histogram of the results of the EIA assay for cGMP levels in C-HUVEC and GD-HUVEC treated and untreated with ovothiol A and in the presence and absence of TNFα and L-NAME (ANOVA test: *p<0.05 vs Basal C-HUVEC, **p<0.05 vs Iono C-HUVEC, #p<0.05 vs Basal GD-HUVEC, 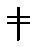 p<0.05 vs Ovothiol C-HUVEC).

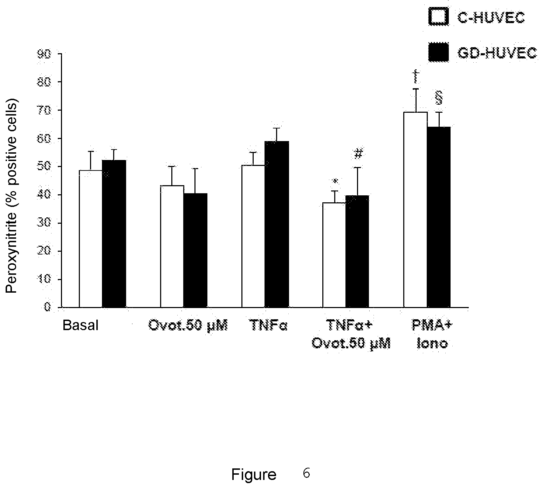

Figure 6 shows the results of experiments performed by flow cytometry using the HK-Green-4A probe for the dosage of peroxynitrite in C-HUVEC and GD-HUVEC treated or not with ovotiolo A and in the presence and absence of TNFα (ANOVA test: *p<0.05 vs TNFα C-HUVEC, #p<0.05 vs TNFα GD-HUVEC, †p<0.05 vs Basal C-HUVEC, §p<0.05 vs Basal GD-HUVEC).

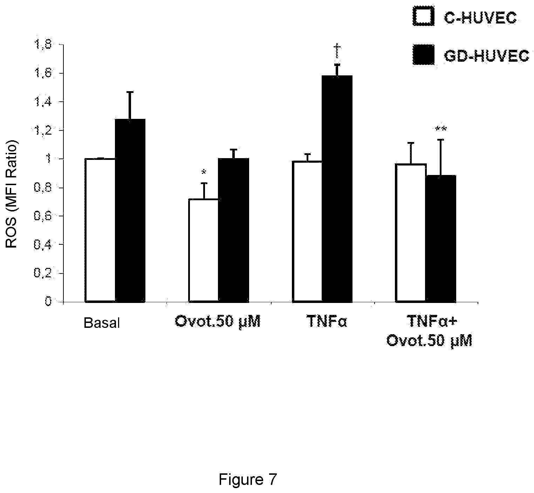

Figure 7 shows the results of flow cytometry for ROS levels in C-HUVEC and GD-HUVEC treated and untreated with ovothiol A and in the presence and absence of TNFα (ANOVA test: *p<0.05 vs Basal C-HUVEC, **p<0.05 vs TNFα GD-HUVEC, †p<0.05 vs TNFα C-HUVEC).

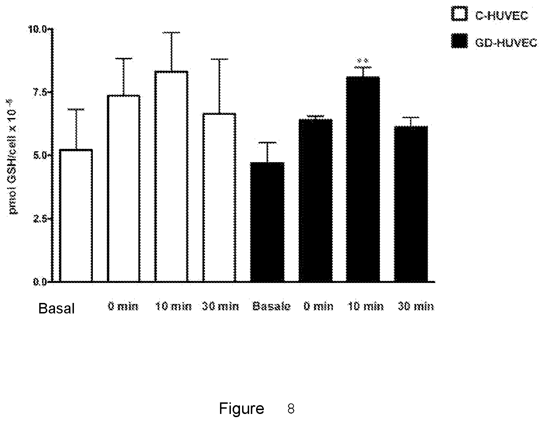

Figure 8 shows the results of intracellular glutathione levels in C-HUVEC and GD-HUVEC treated and untreated with ovothiol A (ANOVA test: **p<0.05 vs Basal).

### Detailed description of the invention

### Definitions

Within the meaning of the present invention, Chronic Low-Grade Systemic Inflammation (CLGSI) means the chronic and non-acute inflammatory state due to environmental and dietary factors, correlated to the lifestyle, that cause metabolic, physical and emotional stress. This leads to the reduction of muscle mass, activation of the hypothalamus-hypophysis-adrenal axis and the sympathetic nervous system with consequent increase in the values of cortisol, angiotensin, adrenaline and noradrenaline, blood glucose that causes hyper-insulinemia and insulin resistance, increased adipose tissue with hypertrophy-hyperplasia of adipose cells that determines the recruitment of macrophages and the production of pro-inflammatory factors, such as interleukins IL2, IL6 and the TNFα factor. It also determines metabolic acidosis, alteration of body composition, reduction of calcium, magnesium, potassium, increase in sodium, and anomalous development of bacterial flora of the oral, gingival and intestinal cavities.

Within the meaning of the present invention, pathologies related to Chronic Low-Grade Systemic Inflammation (CLGSI) means the pathologies included in the group: chronic inflammatory diseases, auto-immune diseases, chronic degenerative diseases, the CLGSI-associated tumors, the obesity, diabetes, arterial hypertension, osteoporosis, respiratory disorders, polycystic ovary syndrome.

Within the meaning of the present invention, the chronic inflammatory diseases are rheumatoid arthritis, polymyalgia rheumatica, fibromyalgia, chronic fatigue syndrome and psoriatic arthritis.

Within the meaning of the present invention, the autoimmune diseases are atopic dermatitis, psoriasis and allergic conjunctivitis.

Within the meaning of the present invention, the chronic degenerative diseases are multiple sclerosis, Parkinson's disease, Amyotrophic lateral sclerosis (ALS) and Alzheimer's disease.

Within the meaning of the present invention, tumours related to Chronic low-grade systemic inflammation (CLGSI) are breast, prostate and colorectal cancers.

Within the meaning of the present invention, diseases of the respiratory apparatus are Allergic rhinitis and Asthma.

The present invention concerns Compounds of formula (I): 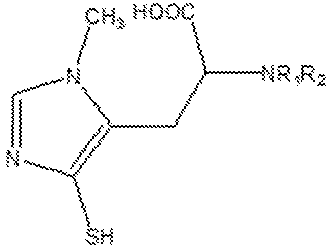 or of formula (II) 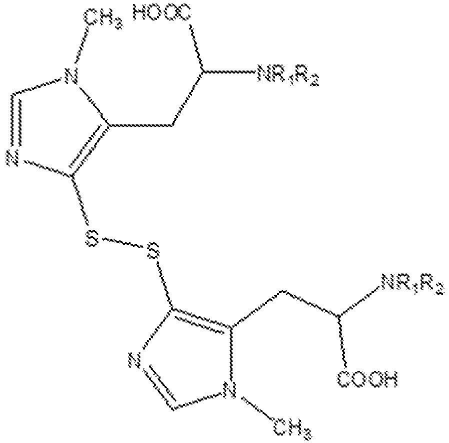 wherein R₁ and R₂, the same or different, are H or CH₃
for the prevention and the treatment of Chronic low-grade systemic inflammation (CLGSI) associated with the diseases related to Chronic low-grade systemic inflammation (CLGSI) selected from the group consisting of: chronic inflammatory diseases, autoimmune diseases, chronic degenerative diseases, tumours related to Chronic low-grade systemic inflammation (CLGSI), obesity, diabetes, arterial hypertension, osteoporosis, diseases of the respiratory apparatus and polycystic ovary syndrome wherein the compounds of formula (I) and (II) reduce chronic low-grade systemic inflammation (CLGSI).

Preferably, the compounds of formula (I) are selected in the group of:
compound of formula(I) wherein R₁ e R₂ are the same and are H (ovothiol A),
compound of formula (I) wherein R₁ is H and R₂ is CH₃ (ovothiol B)
compound of formula (I) wherein R₁ and R₂ are the same and are CH₃ (ovothiol C).

More preferably, compound of formula (I) is ovothiol A.

The compounds of formula (II) are compounds of formula (I) in the oxidized form.

Ovothiol A can be also in the disulphide form of formula (III)

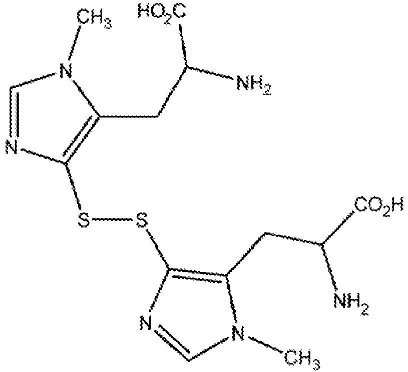

Preferably the diseases related to Chronic low-grade systemic inflammation (CLGSI) are selected from the group consisting of: chronic inflammatory diseases, autoimmune diseases, chronic degenerative diseases, tumours related to Chronic low-grade systemic inflammation (CLGSI), obesity, diabetes, arterial hypertension, osteoporosis, diseases of the respiratory apparatus and polycystic ovary syndrome.

Preferably the chronic inflammatory diseases are selected in the group of: rheumatoid arthritis, polymyalgia rheumatica, fibromyalgia, chronic fatigue syndrome and psoriatic arthritis.

Preferably the autoimmune diseases are selected in the group of: atopic dermatitis, psoriasis and allergic conjunctivitis. Preferably the chronic degenerative diseases are selected in the group of: multiple sclerosis, Parkinson's disease, Amyotrophic lateral sclerosis (ALS) and Alzheimer's disease.

Preferably the tumours related to Chronic low-grade systemic inflammation (CLGSI) are selected in the group of: breast, prostate and colorectal cancers.

Preferably the diseases of the respiratory apparatus are selected in the group of: Allergic rhinitis and Asthma.

Preferably diabetes is of type 2.

### Examples

### Materials and Methods

Preparation of ovothiol A from sea urchin P. *lividus* eggs: ovothiol A was purified as disulphide from P. *lividus* eggs. The animals were collected in the Gulf of Naples, in non-private and unprotected areas, near the city center, by divers, according to the authorization of the Merchant Marine (DPR 1639/68, 09/19/1980 confirmed by D. Lgs. 9/01/2012 n. 4). All animal procedures are in agreement with the guidelines of the European Union (directive 2010/63 and D. Lgs. 4/03/2014 n. 26). The recovered eggs were subjected to a process that involves the fractionation of the aqueous extract without lipids on ion-exchange chromatographic columns and gel filtration (Palumbo, A., d'Ischia, M., Misuraca, G., Prota, G. (1982) Isolation and structure of a new sulphur containing aminoacid from sea urchin eggs. Tetrahedron Lett., 23, 3207-3208). Recently, the procedure has been slightly modified obtaining a yield of 2.5 mg of ovotiolo A for 10 g of eggs (Russo G.L., Russo, M., Castellano, I., Napolitano, A., Palumbo, A. (2014) Ovothiol isolated from sea urchin oocytes induces autophagy in the Hep-G2 cell line. Mar. Drugs, 12, 4069-4085).

Cell endothelial cultures and experimental protocols: umbilical cords were obtained from randomly selected healthy Caucasian (Control, C) and with Gestational Diabetes (GD) mothers delivering at the Hospital of Chieti and Pescara. All procedures were in agreement with the ethical standards of the Institutional Committee on Human Experimentation (Reference Number: 1879/09COET) and with the Declaration of Helsinki Principles. After approval of the protocol by the Institutional Review Board, signed informed consent was obtained from each participating subject. Primary C- and GD-HUVECs were obtained and used between the 3rd and 5th passages *in vitro.* To evaluate the potential cytotoxicity of ovothiol, HUVECs were stimulated for 24h with different doses (10 - 100 µM) of ovothiol A. The best doses of ovothiol A were used to evaluate their effects on the cells treated with TNFα (1ng/ml, Sigma Aldrich) which activated the inflammatory response. Briefly, HUVECs were grown to sub-confluence in complete culture medium DMEM/M199 (Mascia Brunelli, Italia). The cells were pre-incubated with ovothiol A (10 and 50 µM) for 24 hours or with medium alone as basal condition. Subsequently the cells were stimulated with TNFα for 16 hours.

Analysis of Ovothiol A levels in cell cultures: HUVEC cells (1,2 x 10⁶ cellule) were cultured in flasks (25 cm²) in the absence and in the presence of 50 µM ovothiol A and collected after incubation at different time intervals. The culture media were recovered by centrifugation and frozen for ovothiol A quantitation by HPLC analysis. The cellular pellets were resuspended in PBS pH 7.5, sonicated three times for 15 sec and 30 % amplitude. The samples were then centrifuged at 13.000 rpm over 15 min to remove cellular debris, ultrafiltered by Microcon 3 to remove all molecules with MW > 3 kDa and frozen for further analysis by HPLC. HPLC analyses were performed in duplicate on an LC10AD instrument equipped with binary pumps and a Shimadzu SPD-10AVP detector set at 254 nm and 280 nm. A Phenomenex Synergi Sphereclone octadecylsilane (25 cm × 0,46 cm, 4 µm particle size) column was used with 1% formic acid taken to pH 4.5 with ammonia, as the eluant, at a 0.7 mL/min flow rate. Samples exhibiting a peak at the same elution time of standard solutions of ovothiol A were checked by LC/MS run on a LC/MSD Agilent 1100 VL: 0.1% formic acid, pH 4.5 with ammonia, m/z 401 ([M + H]+) for disulfide form and m/z 202 ([M + H]+) for ovothiol A reduced form.

Determination of intracellular glutathione levels (GSH): total GSH was determined by using the Glutathione Assay Kit (Sigma). An aliquot of ultrafiltered samples was added to 3 volumes of 5% 5-sulfosalicylic acid and mixed. Samples were then frozen (-80°C) and thawed at 37°C twice, left for 5 min at 4°C and finally centrifuged at 10.000 g for 10 min. The supernatant was used for the determination of glutathione by performing a kinetic assay in which catalytic amounts (nmoles) of GSH caused a continuous reduction of 5,5-dithiobis (2-nitrobenzoic acid) (DTNB) to 5-thio-2-nitrobenzoic (TNB), measured at 405 nm by a Thermo Scientific™ Multiskan™ FC Microplate Photometer.

MTT assay: ovothiol effect on the viability of C- and GD-HUVECs was evaluated by the 3-(4,5 dimethylthiazolyl-2)-2, 5-diphenyltetrazolium bromide (MTT, Sigma-Aldrich) assay. 1×10⁵ cells were plated in 96-well plates, grown to sub-confluence with the medium alone (as control) or stimulated for 24 h with growing doses of ovothiol A (10-100 µM), in the presence or absence of TNFα stimulation (1ng/ml). A solution of MTT in phosphate buffer saline (PBS) was added to each well to a final concentration of 5 mg/ml. After 3 h incubation at 37°C, the cellular viability was evaluated before addition of 0.2 ml dimethylsulfoxide (DMSO, Sigma-Aldrich) for crystal solubilization in the vital cells. The absorbance at 540 nm was read using a micro-plate reader (SpectraMAX 190, Molecular Devices Inc., Sunnyvale, California, USA) and results were expressed as the absorbance at 540 nm (ABS 540 nm).

Flow cytometry: for flow cytofluorimetry analysis, C- and GD-HUVECs were stimulated as described in the experimental protocols. Non-permeabilized cells were detached by tetraacetic ethylene-diamine acid solution (EDTA) 5 mM, washed and resuspended in bovine serum albumin (BSA) 0.5%. Cells harvested by centrifugation at 800 rpm for 15 min were incubated with anti-VCAM-1 PE-conjugate (1:100, phycoerythrin; Biolegend, San Diego, CA, USA) and anti-ICAM-1 FITC-conjugate (1:100, fluorescein isothiocyanate; Biolegend) antibodies, both for 30 min at 4°C. The intracellular levels of peroxynitrite (ONOO-) were detected in C- and GD-HUVECs by using the HKGreen-4A probe (10 µM, 30 min at 37°C) synthesized and provided by the laboratory of Prof. Dan Yang's. Reactive Oxygen Species (ROS) levels were evaluated with the fluorogenic reagent dichlorofluorescein diacetate (DCF-DA, Sigma-Aldrich) (20 µM, 45 min at 37° C). All samples were analyzed using a FACS Calibur or FACS Canto II (BD Bioscences). The levels of membrane proteins (VCAM-1 and ICAM-1), fluorescence detection of ONOO- and ROS were evaluated by cytometric analysis of over 10.000 events for each sample. All data were analyzed using FACS Diva (BD Bioscences) and FlowJo v.8.8.6 software (TreeStar, Ashland, OR) and expressed as percentage (%) of positive cells or MFI (Mean Fluorescence Intensity) Ratio. The MFI Ratio was calculated by dividing the MFI of positive events by the MFI of negative events (MFI of secondary antibody).

Cyclic guanosine mono phosphate assay (cGMP): intracellular cGMP levels were evaluated by using a commercially available Enzyme Immunoassay (EIA) kit (GE Healthcare, Little Chalfont, Buckinghamshire, UK).

Adhesion of monocytes to endothelium assay: adhesion of U937 monocytes to C- and GD-HUVECs was evaluated in the basal state, after 16 hours of TNFα treatment (1 ng/ml) and in presence of ovothiol A (10-50 µM). The cells were grown to confluence in six-well culture plates and U937 adhesion was evaluated as previously described (Di Tomo, P., Lanuti, P., Di Pietro, N., Baldassarre, M.P.A., Marchisio, M., Pandolfi, A., Consoli, A., Formoso, G. (2017) Liraglutide mitigates TNF-α induced pro-atherogenic changes and Microvesicles release in HUVEC from diabetic women. Diabetes Metab. Res. Rev. doi: 10.1002/dmrr.2925).

Statistical analysis: all experiments were repeated at least three times employing different cell strains of C- e GD-HUVECs and the results are presented as means ± standard deviation (SD). Differences between the two cell strains and between the different treatments were analyzed by Student's t-test and one-way analysis of variance (ANOVA) followed by Bonferroni multiple comparison test for post hoc comparisons.

In order to ensure the validity of the chosen experimental system, the viability of C-HUVECs and GD-HUVECs was first evaluated by MTT assay. Since at the concentration of 100 µM ovothiol A was cytotoxic in the cellular model tested (as shown in figure 1A), the concentration of 50 µM was selected to carry out the subsequent assays. The C- and GD-HUVEC cells treated with 50 µM of ovothiol A in the presence or absence of TNFα showed no significant changes in the cell viability parameters, as shown in Figure 1B.

Subsequently, the bioavailability of ovothiol A for endothelial cells was evaluated. C- and GD-HUVECs were incubated for 30 minutes with ovothiol A (50 µM) and the presence of the molecule in the culture medium and within cells was determined by HPLC. The kinetic decay of the compound in the medium of either cell types was very rapid, as shown in Figure 2, compared to the medium without the cells. However, molecule absorption is slightly more effective in GD-HUVECs compared to C-HUVECs, as the amount found within cells in the first 30 minutes is higher in GD-HUVECs than in C-HUVECs (1,53 pmol/cell vs 1,18 pmol/cell), as shown in Table 1.

**Table 1**

| | medium | cells |
|---|---|---|
| | (µmol/L) | (pmol/cell) |
| Control | 45 | |
| C-HUVECs | 14.86 ±1,24 | 1.18 |
| GD-HUVECs | 10.08 ±1,68 | 1.53 |

Later, the effect of ovothiol A on the adhesion of human monocytes to the endothelium was investigated. Human endothelial cells GD-HUVEC and related controls (C-HUVEC) were incubated for 24 hours with ovothiol A (10-50 µM) and stimulated with TNFα (1 ng/ml) for 16 hours to induce the inflammation. U937 human monocytes adhesion to endothelium was measured, counting the number of U937 adherent cells on the HUVEC monolayer, since it represents an early event in the development of atherosclerosis. As shown in Figure 3, the number of adherent cells on both types of HUVEC monolayers increased dramatically after TNFα stimulation. After 24 hours of pre-treatment with 10 and 50 µM of ovothiol A a significant reduction of cell adhesion was observed only on GD-HUVECs. As expected, when C- and GD-HUVECs were treated with antibodies against VCAM-1 or ICAM-1 at saturating concentrations 1 hour before the assay, U937 adhesion was suppressed in both cell types, suggesting that VCAM-1 and ICAM-1 hyper-expression on the cell surface plays a crucial role for increased monocytes adhesion to HUVECs.

Furthermore, membrane exposure levels of vascular (VCAM-1) and intercellular (ICAM-1) cell adhesion molecule were evaluated by flow cytometry. Figure 4 shows increased exposure levels of VCAM-1 and ICAM-1 in both endothelial cell models after treatment with TNFα (p <0.05). Ovothiol A significantly reduced the exposure of VCAM-1 on the membrane in both C-HUVEC and GD-HUVEC cells, while the effect on ICAM-1 levels did not reach the statistical significance.

As shown in Figure 5, the bioavailability of nitric oxide (NO) was evaluated by determining the cGMP levels with EIA assay. Following ovothiol A treatment, cGMP levels significantly increased in C- but not in GD-HUVECs and this effect was abolished by TNFα treatment (TNFα+Ovot). The effect of ovothiol A was not affected by pre-incubation with L-NAME (1 mM for 45 minutes), a known inhibitor of nitric oxide synthase (NOS), thus suggesting that NO production is not derived from the modulation of the enzymatic activity of eNOS but by the reduction of oxidative stress. As expected, in C-HUVECs the positive control Ionomycin (Iono) induced the increase of cGMP levels and the addition of L-NAME restored the initial levels.

The levels of peroxynitrite were evaluated by flow cytometry using HKGreen-4A probe. After exposure to TNFα, the pre-treatment with 50 µM ovothiol A significantly reduced the levels of peroxynitrite, a marker of nitro-oxidative stress, in both cultures. As positive control, Ionomycin (Iono) 50 nM in combination with phorbol 12-myristate 13-acetate (PMA) 0,3 µM induced peroxynitrite formation in both HUVEC cells, as shown in Figure 6.

Subsequently, ROS levels in C- and GD-HUVECs were assessed in the absence and presence of TNFα. The addition of ovothiol A reduced ROS levels in C-HUVECs in the absence of TNFα and in GD-HUVECs under inflammatory conditions, as shown in Figure 7.

Further, the intracellular levels of glutathione were measured in the first stage of absorption of ovothiol by the GD-HUVECs compared to C-HUVECs. As shown in Figure 8, intracellular GSH levels increased significantly after 10 minutes of incubation of ovothiol A, at least in GD-HUVECs, suggesting a redox exchange with glutathione.

## Claims

1. Compounds of formula (I): 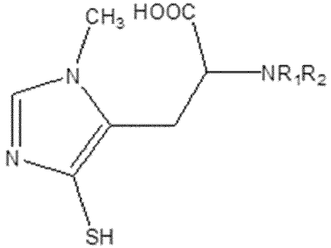 or of formula (II) 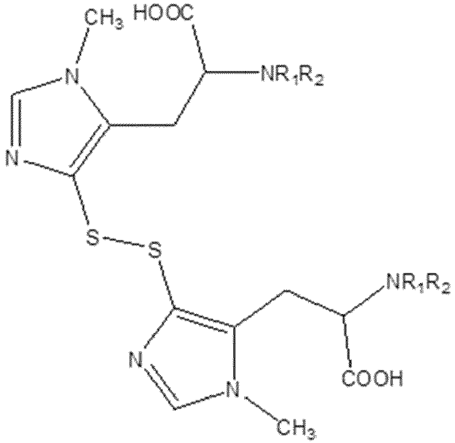 wherein R₁ and R₂, the same or different, are H or CH₃
for use for the prevention and the treatment of Chronic low-grade systemic inflammation (CLGSI) associated with the diseases related to Chronic low-grade systemic inflammation (CLGSI) selected from the group consisting of: chronic inflammatory diseases, autoimmune diseases, chronic degenerative diseases, tumours related to Chronic low-grade systemic inflammation (CLGSI), obesity, diabetes, arterial hypertension, osteoporosis, diseases of the respiratory apparatus and polycystic ovary syndrome wherein the compounds of formula (I) and (II) reduce chronic low-grade systemic inflammation.

2. Compounds for use for the prevention and the treatment of Chronic low-grade systemic inflammation (CLGSI) and the diseases related to Chronic low-grade systemic inflammation (CLGSI) according to claim 1 selected from the group consisting of:
R₁ and R₂ are the same and are H,
R₁ is H and R₂ is CH₃,
R₁ and R₂ are the same and are CH₃.

3. Compounds for use for the prevention and the treatment of Chronic low-grade systemic inflammation (CLGSI) and the diseases related to Chronic low-grade systemic inflammation (CLGSI) according to claim 2 wherein R₁ and R₂ are the same and are in the form of disulfide of formula (III)

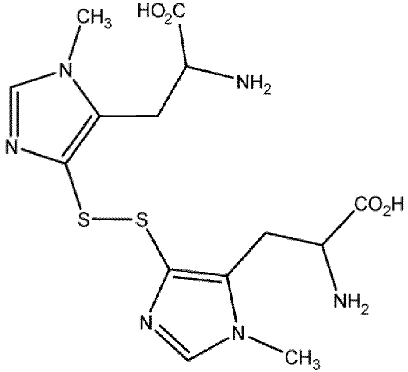

4. Compounds for use for the prevention and the treatment of Chronic low-grade systemic inflammation (CLGSI) and the diseases related to Chronic low-grade systemic inflammation (CLGSI) according to claim 1 wherein chronic inflammatory diseases are selected from the group consisting of: rheumatoid arthritis, polymyalgia rheumatica, fibromyalgia, chronic fatigue syndrome and psoriatic arthritis.

5. Compounds for use for the prevention and the treatment of Chronic low-grade systemic inflammation (CLGSI) and the diseases related to Chronic low-grade systemic inflammation (CLGSI) according to claim 1 wherein autoimmune diseases are selected from the group consisting of: atopic dermatitis, psoriasis and allergic conjunctivitis.

6. Compounds for use for the prevention and the treatment of Chronic low-grade systemic inflammation (CLGSI) and the diseases related to Chronic low-grade systemic inflammation (CLGSI) according to claim 1 wherein chronic degenerative diseases are selected from the group consisting of: multiple sclerosis, Parkinson's disease, Amyotrophic lateral sclerosis (ALS) and Alzheimer's disease.

7. Compounds for use for the prevention and the treatment of Chronic low-grade systemic inflammation (CLGSI) and the diseases related to Chronic low-grade systemic inflammation (CLGSI) according to claim 1 wherein tumours related to Chronic low-grade systemic inflammation (CLGSI) are selected from the group consisting of: breast cancer, prostate cancer and colorectal cancer.

8. Compounds for use for the prevention and the treatment of Chronic low-grade systemic inflammation (CLGSI) and the diseases related to Chronic low-grade systemic inflammation (CLGSI) according to claim 1 wherein diseases of the respiratory apparatus are selected from the group consisting of: Allergic rhinitis and Asthma.

9. Compounds for use for the prevention and the treatment of Chronic low-grade systemic inflammation (CLGSI) and the diseases related to Chronic low-grade systemic inflammation (CLGSI) according to claim 1 wherein diabetes is type 2 diabetes.

## Patentansprüche

1. Verbindungen der Formel (I): 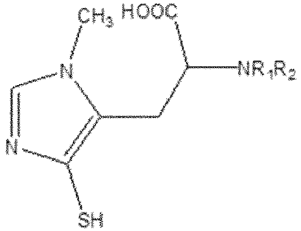 oder der Formel (II) 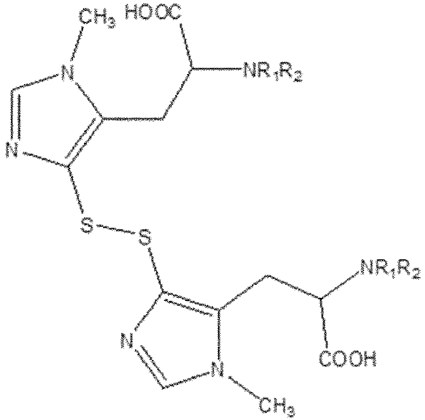 worin R₁ und R₂, die gleich oder verschieden sind, H oder CH₃ sind,
zur Verwendung für die Prävention und die Behandlung von chronischer niedrigschwelliger systemischer Entzündung (CLGSI), verbunden mit den Krankheiten, die mit chronischer niedrigschwelliger systemischer Entzündung (CLGSI) in Beziehung stehen, ausgewählt aus der Gruppe, bestehend aus: chronischen Entzündungskrankheiten, Autoimmunkrankheiten, chronischen degenerativen Krankheiten, Tumoren, die mit chronischer niedrigschwelliger systemischer Entzündung (CLGSI) in Beziehung stehen, Obesitas, Diabetes, arterieller Hypertonie, Osteoporose, Krankheiten der Atemwege und polyzystischem Ovarialsyndrom, worin die Verbindungen der Formel (I) und (II) chronische niedrigschwellige systemische Entzündung reduzieren.

2. Verbindungen zur Verwendung für die Prävention und die Behandlung von chronischer niedrigschwelliger systemischer Entzündung (CLGSI) und der Krankheiten, die mit chronischer niedrigschwelliger systemischer Entzündung (CLGSI) in Beziehung stehen, gemäß Anspruch 1, ausgewählt aus der Gruppe, bestehend aus:
R₁ und R₂ sind gleich und sind H,
R₁ ist H und R₂ ist CH₃,
R₁ und R₂ sind gleich und sind CH₃.

3. Verbindungen zur Verwendung für die Prävention und die Behandlung von chronischer niedrigschwelliger systemischer Entzündung (CLGSI) und der Krankheiten, die mit chronischer niedrigschwelliger systemischer Entzündung (CLGSI) in Beziehung stehen, gemäß Anspruch 2, worin R₁ und R₂ gleich sind und in Form des Disulfids nach Formel (III) vorliegen

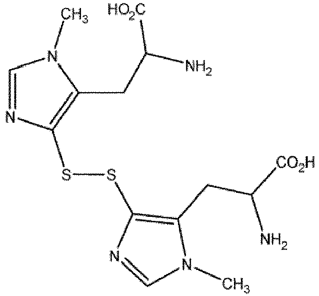

4. Verbindungen zur Verwendung für die Prävention und die Behandlung von chronischer niedrigschwelliger systemischer Entzündung (CLGSI) und der Krankheiten, die mit chronischer niedrigschwelliger systemischer Entzündung (CLGSI) in Beziehung stehen, gemäß Anspruch 1, worin die chronischen entzündlichen Krankheiten ausgewählt sind aus der Gruppe, bestehend aus: rheumatoider Arthritis, Polymyalgia Rheumatica, Fibromyalgie, chronischem Erschöpfungssyndrom und Psoriasis-Arthritis.

5. Verbindungen zur Verwendung für die Prävention und die Behandlung von chronischer niedrigschwelliger systemischer Entzündung (CLGSI) und der Krankheiten, die mit chronischer niedrigschwelliger systemischer Entzündung (CLGSI) in Beziehung stehen, gemäß Anspruch 1, worin die Autoimmunkrankheiten ausgewählt sind aus der Gruppe, bestehend aus: atopischer Dermatitis, Psoriasis und allergischer Konjunktivitis.

6. Verbindungen zur Verwendung für die Prävention und die Behandlung von chronischer niedrigschwelliger systemischer Entzündung (CLGSI) und der Krankheiten, die mit chronischer niedrigschwelliger systemischer Entzündung (CLGSI) in Beziehung stehen, gemäß Anspruch 1, worin die chronischen degenerativen Krankheiten ausgewählt sind aus der Gruppe, bestehend aus: multipler Sklerose, Morbus Parkinson, amyotropher Lateralsklerose (ALS) und Morbus Alzheimer.

7. Verbindungen zur Verwendung für die Prävention und die Behandlung von chronischer niedrigschwelliger systemischer Entzündung (CLGSI) und der Krankheiten, die mit chronischer niedrigschwelliger systemischer Entzündung (CLGSI) in Beziehung stehen, gemäß Anspruch 1, worin Tumore, die mit chronischer niedrigschwelliger systemischer Entzündung (CLGSI) in Beziehung stehen, ausgewählt sind aus der Gruppe, bestehend aus: Brustkrebs, Prostatakrebs und Darmkrebs.

8. Verbindungen zur Verwendung für die Prävention und die Behandlung von chronischer niedrigschwelliger systemischer Entzündung (CLGSI) und der Krankheiten, die mit chronischer niedrigschwelliger systemischer Entzündung (CLGSI) in Beziehung stehen, gemäß Anspruch 1, wobei die Krankheiten der Atemwege ausgewählt sind aus der Gruppe, bestehend aus: allergischer Rhinitis und Asthma.

9. Verbindungen zur Verwendung für die Prävention und die Behandlung von chronischer niedrigschwelliger systemischer Entzündung (CLGSI) und der Krankheiten, die mit chronischer niedrigschwelliger systemischer Entzündung (CLGSI) in Beziehung stehen, gemäß Anspruch 1, wobei der Diabetes Typ-2-Diabetes ist.

## Revendications

1. Composés de formule (I) : 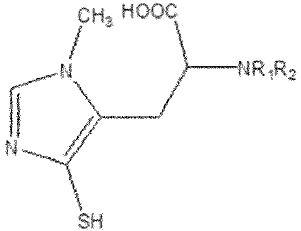 ou de formule (II) 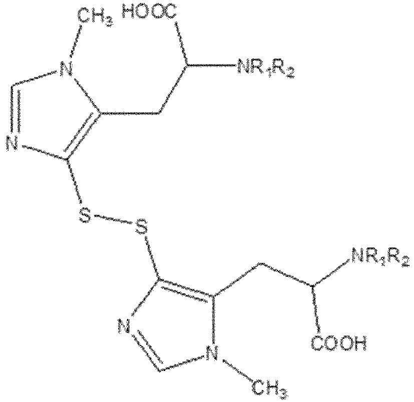 dans lesquels R₁ et R₂, identiques ou différents, sont H ou CH₃
pour leur utilisation dans la prévention et le traitement d'une inflammation systémique chronique de bas grade (CLGSI) associée aux maladies liées à une inflammation systémique chronique de bas grade (CLGSI) sélectionnées dans le groupe consistant en : des maladies inflammatoires chroniques, des maladies auto-immunes, des maladies dégénératives chroniques, des tumeurs liées à une inflammation systémique chronique de bas grade (CLGSI), une obésité, un diabète, une hypertension artérielle, une ostéoporose, des maladies de l'appareil respiratoire et un syndrome des ovaires polykystiques dans lesquels les composés de formule (I) et (II) réduisent une inflammation systémique chronique de bas grade.

2. Composés pour leur utilisation dans la prévention et le traitement d'une inflammation systémique chronique de bas grade (CLGSI) et des maladies liées à une inflammation systémique chronique de bas grade (CLGSI) selon la revendication 1 sélectionnés dans le groupe consistant en :
R₁ et R₂ sont identiques et sont H,
R₁ est H et R₂ est CH₃,
R₁ et R₂ sont identiques et sont CH₃.

3. Composés pour leur utilisation dans la prévention et le traitement d'une inflammation systémique chronique de bas grade (CLGSI) et des maladies liées à une inflammation systémique chronique de bas grade (CLGSI) selon la revendication 2 dans lesquels R₁ et R₂ sont identiques et sont sous la forme d'un disulfure de formule (III)

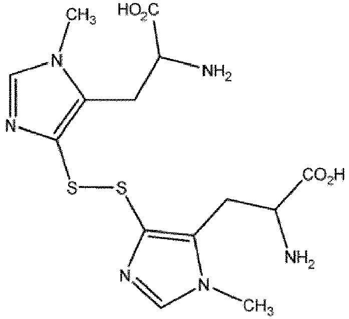

4. Composés pour leur utilisation dans la prévention et le traitement d'une inflammation systémique chronique de bas grade (CLGSI) et des maladies liées à une inflammation systémique chronique de bas grade (CLGSI) selon la revendication 1 dans lesquels les maladies inflammatoires chroniques sont sélectionnées dans le groupe consistant en : une polyarthrite rhumatoïde, une polymyalgie rhumatismale, une fibromyalgie, un syndrome de fatigue chronique et une polyarthrite psoriasique.

5. Composés pour leur utilisation dans la prévention et le traitement d'une inflammation systémique chronique de bas grade (CLGSI) et des maladies liées à une inflammation systémique chronique de bas grade (CLGSI) selon la revendication 1 dans lesquels les maladies auto-immunes sont sélectionnées dans le groupe consistant en : une dermatite atopique, un psoriasis et une conjonctivite allergique.

6. Composés pour leur utilisation dans la prévention et le traitement d'une inflammation systémique chronique de bas grade (CLGSI) et des maladies liées à une inflammation systémique chronique de bas grade (CLGSI) selon la revendication 1 dans lesquels les maladies dégénératives chroniques sont sélectionnées dans le groupe consistant en : une sclérose en plaques, une maladie de Parkinson, une sclérose latérale amyotrophique (SLA) et une maladie d'Alzheimer.

7. Composés pour leur utilisation dans la prévention et le traitement d'une inflammation systémique chronique de bas grade (CLGSI) et des maladies liées à une inflammation systémique chronique de bas grade (CLGSI) selon la revendication 1 dans lesquels les tumeurs liées à une inflammation systémique chronique de bas grade (CLGSI) sont sélectionnées dans le groupe consistant en : un cancer du sein, un cancer de la prostate et un cancer colorectal.

8. Composés pour leur utilisation dans la prévention et le traitement d'une inflammation systémique chronique de bas grade (CLGSI) et des maladies liées à une inflammation systémique chronique de bas grade (CLGSI) selon la revendication 1 dans lesquels les maladies de l'appareil respiratoire sont sélectionnées dans le groupe consistant en : une rhinite allergique et un asthme.

9. Composés pour leur utilisation dans la prévention et le traitement d'une inflammation systémique chronique de bas grade (CLGSI) et des maladies liées à une inflammation systémique chronique de bas grade (CLGSI) selon la revendication 1 dans lesquels le diabète est le diabète de type 2.
